# EUROPEAN PATENT SPECIFICATION

(11) **EP 0 631 668 B1**
(45) Date of publication and mention of the grant of the patent: **04.09.2002**
(21) Application number: 93907499.3
(22) Date of filing: 15.03.1993
(51) Int. Cl.: G01N 33/553, G01N 33/543, B03C 1/01

(54) **DETERMINATION OF BINDING AFFINITY USING MAGNETICALLY-LABELED BINDING MEMBERS**
BESTIMMUNG VON BINDUNGSAFFINITÄT MITTELS MAGNETISCH MARKIERTER BINDUNGSPARTNER
DETERMINATION DE L'AFFINITE DE LIAISON METTANT EN OEUVRE DES ELEMENTS DE LIAISON MARQUES MAGNETIQUEMENT

(30) Priority: 20.03.1992 US 854186
(43) Date of publication of application: 04.01.1995
(73) Proprietor: ABBOTT LABORATORIES, Abbott Park, Illinois 60064-3500 (US)
(72) Inventor: ROHR, Thomas, E., Gurnee, IL 60031 (US)
(74) Representative: Modiano, Guido, Dr.-Ing.
(86) International application number: US9302337
(87) International publication number: WO93019371

(56) References cited:
- WO-A-88/02118
- WO-A-93/19370

## Description

### BACKGROUND OF THE INVENTION

### 1. Field of the Invention

This invention relates to a method for determining the association constant between first and second binding pair members. In particular, the invention relates to the use of magnetically attractable materials as the detectable label.

### 2. Description of Related Art

Diagnostic assays have become an indispensable means for detecting analytes in test samples by using the mutual reaction between the analyte and a specific binding member, as typified by the immunoreaction between an antigen and the antibody to that antigen. In detecting binding reactions, use has been made of detectable tags or labels attached to antibodies, which in turn bind to the analytes of interest. The detection of the resultant labeled antibody/analyte complex, or of the labeled antibody which remains unbound, is used to indicate the presence or amount of the analyte in the test sample.

Two commonly used binding assay techniques are the radioimmunoassay (RIA) and the enzyme immunoassay (EIA), both of which employ a labeled binding member. The RIA uses a radioactive isotope as the traceable substance attached to a binding member. Because the radioactive isotope can be detected in very small amounts, it can be used to detect or quantitate small amounts of analyte. There are, however, a number of substantial drawbacks associated with the RIA. These drawbacks include the special facilities and extreme caution that are required in handling radioactive materials, the high costs of such reagents and their unique disposal requirements.

The EIA uses an enzyme as the label attached to a binding member, and enzymatic activity is used to detect the immunoreaction. While the EIA does not have the same disadvantages as the RIA, EIA techniques typically require the addition of substrate materials to elicit the detectable enzyme reaction. Enzyme substrates are also often unstable and have to be prepared just prior to use or be stored under refrigeration. In addition, enzyme labels may be difficult to purify and conjugate to binding members, and may be unstable during storage at room temperature. Enzyme immunoassays are also unsatisfactory in that the methods typically require complex incubations, multiple liquid additions and multiple wash steps.

More recently, assay techniques using metallic sol particles as visual labels have been developed. In these techniques, a metal (e.g., gold, silver, platinum), a metal compound, or a nonmetallic substance coated with a metal or a metal compound is used to form an aqueous dispersion of particles. Generally, the binding member to be labeled is coated onto the metal sol particles by adsorption, and the particles are captured or aggregated in the presence of analyte. The metal sol particles have the advantage of producing a signal that is visually detectable as well as measurable by an instrument, but despite their utility, the inorganic particles have several disadvantages. Metallic particles are difficult to quantitatively measure. The metallic particles also have a limited color intensity, and therefore, limited sensitivity in some assays. In addition, the surfaces of inorganic metallic colloid particles, such as gold, do not readily accept the covalent attachment of binding members. Thus, during use in a binding assay, care must be taken so that the adsorbed binding members are not removed from the inorganic particles through the combination of displacement by other proteins or surface active agents and the shear forces which accompany washing steps used to remove non-specifically bound material. Sol particles can be difficult to coat without inducing aggregation, may aggregate upon storage and may aggregate upon the addition of buffers or salts. Furthermore, such particulate labels are difficult to concentrate, may aggregate during use and are difficult to disperse.

Other label materials include chemiluminescent and fluorescent substances. Non-metallic particles, such as dyed or colored latex and selenium particles have also been used as visual labels.

The use of magnetic particles in binding assays is known, but prior to the present invention magnetic particles have generally been used as a means to remove or sequester the analyte component of the test sample. For example, U.S. Patent Nos. 4,070,246 and 3,985,649 teach the use of binding members attached to ferromagnetic particles, whereby the binding member forms a complex with the analyte of interest, and the resulting complex is removed from the reaction mixture by means of a magnetic field. Alternatively, Hersh et al. (U.S. Patent No. 3,933,997) teach the use of magnetic particles as a means of concentrating a radioactive material on a test substance. Ebersole (U.S. Patent No. 4,219,335) teaches the use of magnetic particles which have characteristics capable of affecting electrical resistance, wherein a capacitance measurement will reveal whether the particles are present on a surface.

### SUMMARY OF THE INVENTION

The present invention advantageously uses a magnetically attractable material as a detectable label in a method for determining the association constant between members of a binding pair. The magnetic responsiveness of the magnetically-labeled reagent results in the exertion of a force upon the labeled reagent and a solid phase. The measurement of the changes in force exerted by a magnetic field upon the solid phase indicates the association constant between a first and a second binding member.

In brief, the method involves reacting a first and a second binding member thereby forming a binding pair complex, wherein one binding member is attached to a solid phase and one binding member is attached to a magnetically attractable label to form a magnetically-labeled reagent. Unbound magnetically-labeled reagent is separated from that magnetically-labeled reagent which has bound to a solid phase, and a magnetic field is applied to the bound magnetically-labeled reagent. The resultant force exerted upon the solid phase is measured. The magnetic field applied to the bound magnetically-labeled reagent is then increased to a level sufficient to dissociate at least a portion of the bound magnetically-labeled reagent from the solid phase. The resultant change in force exerted upon the solid phase is then detected, whereby that change indicates the association constant between the first and second binding members.

The extent of the force exerted by the magnetic field upon the magnetically responsive label or the solid phase may be determined by observing an apparent weight change of the bound reagent, by determining the displacement of the bound reagent, by determining the mass change of the label or by determining the movement of the bound reagent in the magnetic field. The detection means for such observations may involve weighing or balance means, motion detectors, pressure sensors, position sensors, optical sensors, frequency sensors, etc. Any means suitable for detecting the magnetic responsiveness of the bound or unbound magnetically-labeled reagent can be used.

### DESCRIPTION OF THE DRAWINGS

Figure 1 is a schematic view of the magnetically assisted detection of magnetically-labeled reagent using a balance or weighing means.
Figure 2 is a schematic view of a balance means in operation for the magnetically assisted detection of a magnetically-labeled reagent.
Figure 3 is a schematic view of the magnetically assisted detection of a magnetically-labeled reagent using an optical sensor means.
Figure 4 is a schematic view of an optical sensor means in operation for the magnetically assisted detection of a magnetically-labeled reagent.
Figure 5 illustrates the measurement of the attractive force of unbound or free magnetically-labeled reagent.
Figure 6 illustrates the results of a binding assay using a magnetically-labeled reagent as the detectable label.
Figure 7 illustrates the results of a binding assay using a magnetically-labeled reagent as the detectable label, plotted as an inhibition curve.
Figure 8 depicts the effect of the repeated approach and withdrawal of a magnetic field from a solid phase containing antibody-coated magnetic particles captured by an immobilized antibody.
Figure 9 depicts a single cycle of the approach and withdrawal of a magnetic field from a solid phase containing antibody-coated magnetic particles captured by an immobilized antibody.
Figure 10 depicts the decrease in weight change due to the presence of free antibody during incubation in a system as illustrated in Figure 9.
Figure 11 depicts an inhibition curve from a magnetically assisted immunoassay.
Figure 12 depicts a schematic view of the measurement means for the determination of the strength of associations between complementary binding members.
Figure 13 depicts an instrument tracing of the weight changes resulting from the approach of a magnet to the top of a vessel containing an immobilized magnetically-labeled reagent.

### DETAILED DESCRIPTION OF THE INVENTION

When a magnetically responsive material is placed under the influence of a magnetic field, the material will tend to move towards or away from the region where the magnetic field is the strongest. For example, a paramagnetic material, such as ferrite, will be attracted to the magnetic field while a diamagnetic material, such as polystyrene, will move away from the magnetic field. The force of the response or the movement of such magnetically responsive material may be viewed as a measure of the amount of material present. The present invention results from the surprising discovery that when a magnetically responsive material is used as a label in a binding assay, it is possible to detect the presence or amount of either the free or unbound label by measuring the aggregate force exerted upon the labels by an applied magnetic field. Furthermore, the strength of the detectable force bears a definite relationship to the amount of the bound or free magnetically attractable label, thereby permitting a determination of the presence or amount of the analyte in the test sample. The discovery has several important consequences.

Conventional heterogeneous binding assay formats require vigorous washing of the solid phase to separate bound and unbound labeled reagent and to suppress the nonspecific binding of materials to the solid phase. Such wash steps complicate the assay protocol and restrict the assay to the use of binding pair members having high affinity, i.e., a binding strength that will withstand such physical manipulation. In one aspect, the present invention avoids the need for complex washing steps in binding assays because unbound or non-specifically bound label can be separated from the reaction mixture by the application of a first magnetic field prior to the detection of specifically bound label by means of a second magnetic field. The high degree of control that is possible over the magnetic field permits the use of a first field that is suitable to separate free or non-specifically bound label from a reaction mixture without affecting specifically bound label. In turn, this permits the use of lower affinity binding members whose binding will not be significantly affected by the first magnetic field.

In conventional particle agglutination assays, binding members of low affinity can be used because several binding sites on each member can cooperate to give high avidities, and the absence of wash steps allows weak associations to be maintained while simplifying the assay format. Signal amplification results from the fact that the interaction of a few binding sites can cause the aggregation of binding members several orders of magnitude greater in size and mass than the original members, and thereby provide a macroscopic change which can be interpreted visually. Unfortunately, particle agglutination assays are often difficult to interpret, do not yield quantitative results, and are not readily amenable to automation.

The present invention avoids these problems by placing the magnetic label in a magnetic field, and measuring the magnetic force exerted upon the label to provide a qualitative or quantitative assay readout. The force affect of the magnetic field upon the magnetic label enhances the detection of the captured or aggregated magnetic label while suppressing non-specific interference from any non-magnetic substances. Force enhancements approaching three orders of magnitude have been achieved by the application of a magnetic field and the detection of the resultant force affect on the magnetic label in that field. In comparison to the detection of weight changes due to binding reactions (e.g., the detection of the weight of bound analyte as is determined in conventional gravimetric analyses) the present invention provides binding assay signal enhancements of nine orders of magnitude and is sufficient to detect analyte concentrations in the femtomolar (10⁻¹⁵ mole or one quadrillionth mole) range.

In another aspect, the intensity of the magnetic field can be precisely manipulated, e.g., by means of an electromagnetic or a movable permanent magnet. A field intensity can be chosen which is optimal for a particular assay and selected binding reagents, such that a field sufficient to remove unbound and non-specifically bound magnetic label can be applied without disrupting the associations formed between the binding members. This provides the opportunity to use binding members having lower binding affinities than those typically found useful in binding assays.

In another aspect, the nonspecific binding of extraneous substances to the solid phase will not interfere with the analyte determination. Only the force affect of the magnetic label in a magnetic field is measured, as opposed to determining the total weight of the resulting binding complex or reagents upon the solid phase.

In another aspect, small levels of force can be readily determined using detection means which include, but are not limited to, electronic balances, optical sensors, piezoelectric pressure sensing devices (including micromechanical silicon devices or electronic chips), vibrating fiber devices and cantilever beam devices (including those used to sense force changes in an atomic force microscope). This enables very sensitive assays and obviates the need for amplification of the label as required in many conventional assays.

In yet another aspect, these advantages permit the assays to be readily adapted to computer control. Therefore, the present invention is suitable for automated systems.

In yet another aspect, the intensity of the magnetic field can be precisely manipulated to disrupt the associations formed between the binding members. Thus, the present invention also provides a means to evaluate the binding affinities or association constants of binding members.

Before proceeding with the description of the various embodiments of the present invention, a number of terms used herein will be defined.

### Definitions

The following definitions are applicable to the present invention.

The term "test sample", as used herein, refers to a material suspected of containing the analyte. The test sample can be used directly as obtained from the source or following a pretreatment to modify the character of the sample. The test sample can be derived from any biological source, such as a physiological fluid, including, blood, saliva, ocular lens fluid, cerebral spinal fluid, sweat, urine, milk, ascites fluid, mucous, synovial fluid, peritoneal fluid, amniotic fluid or the like. The test sample can be pretreated prior to use, such as preparing plasma from blood, diluting viscous fluids, or the like; methods of treatment can involve filtration, distillation, concentration, inactivation of interfering components, and the addition of reagents such as buffers and extraction reagents. Besides physiological fluids, other liquid samples can be used such as water, food products and the like for the performance of environmental or food production assays. In addition, a solid material suspected of containing the analyte can be used as the test sample. In some instances it may be beneficial to modify a solid test sample to form a liquid medium or to release the analyte.

The term "binding member", as used herein, refers to a member of a binding pair, i.e., two different molecules wherein one of the molecules specifically binds to the second molecule through chemical or physical means. In addition to the well-known antigen and antibody binding pair members, other binding pairs include, as examples without limitation, biotin and avidin, carbohydrates and lectins, complementary nucleotide sequences, complementary peptide sequences, effector and receptor molecules, enzyme cofactors and enzymes, enzyme inhibitors and enzymes, a peptide sequence and an antibody specific for the sequence or the entire protein, polymeric acids and bases, dyes and protein binders, peptides and specific protein binders (e.g., ribonuclease, S-peptide and ribonuclease S-protein), sugar and boronic acid, and similar molecules having an affinity which permits their association in a binding assay. Furthermore, binding pairs can include members that are analogs of the original binding member, for example an analyte-analog or a binding member made by recombinant techniques or molecular engineering. If the binding member is an immunoreactant it can be, for example, an antibody, antigen, hapten, or complex thereof, and if an antibody is used, it can be a monoclonal or polyclonal antibody, a recombinant protein or antibody, a chimeric antibody, a mixture(s) or fragment(s) thereof, as well as a mixture of an antibody and other binding members. The details of the preparation of such antibodies, peptides and nucleotides and their suitability for use as binding members in a binding assay are well-known to those skilled-in-the-art.

The term "analyte" or "analyte of interest", as used herein, refers to the compound or composition to be detected or measured and which has at least one epitope or binding site. The analyte can be any substance for which there exists a naturally occurring binding member or for which a binding member can be prepared. Analytes include, but are not limited to toxins, organic compounds, proteins, peptides, microorganisms, amino acids, nucleic acids, hormones, steroids, vitamins, drugs (including those administered for therapeutic purposes as well as those administered for illicit purposes), and metabolites of or antibodies to any of the above substances. The term "analyte" also includes any antigenic substances, haptens, antibodies, macromolecules and combinations thereof.

The term "analyte-analog", as used herein, refers to a substance which cross-reacts with an analyte-specific binding member, although it may do so to a greater or a lesser extent than does the analyte itself. The analyte-analog can include a modified analyte as well as a fragmented or synthetic portion of the analyte molecule, so long as the analyte-analog has at least one epitopic site in common with the analyte of interest. An example of an analyte-analog is a synthetic peptide sequence which duplicates at least one epitope of the whole-molecule analyte so that the analyte-analog can bind to an analyte-specific binding member.

The term "magnetically-labeled reagent", as used herein, refers to a substance involving a magnetically attractable label attached to a binding member. The attachment may be affected by covalent or non-covalent binding means, linking arms, etc., but the method of attachment is not critical to the present invention. Upon the application of a magnetic field, the magnetically attractable label allows the reagent to produce a detectable response that will be directly or indirectly related to the amount of analyte in the test sample. The binding member component of the reagent may be selected to directly bind the analyte or to indirectly bind the analyte by means of an ancillary specific binding member, which is described in greater detail hereinafter. Magnetically-labeled reagents include binding members that are attached to a magnetically attractable label before, during or after incubation with the test sample and/or other assay reagents. The terms "binding member attached to a magnetically attractable particle","binding member attached to a magnetic material", "binding member attached to a magnetic label", "binding member attached to a magnetically responsive label" and similar terms are interchange and are used to refer to the main characteristic of the magnetically-labeled reagents of the present invention, i.e., the label produces a detectable response when placed in the vicinity of a magnetic field.

The selection of a particular composition of magnetic label material is not critical to the present invention, but several requirements do apply. The magnetically attractable material must bind, carry or be modifiable so as to attach to a binding member which will in turn bind another assay reagent or a component present in the test sample. The label must be magnetically attractable, preferably to an extent which permits both the rapid removal or separation of unbound magnetically-labeled reagent and the production of a detectable magnetic force upon exposure to a magnetic field. For the purposes of the present invention, a material is magnetically responsive if it is influenced by the application of a magnetic field, for example, it is attracted or repulsed or has a detectable magnetic susceptibility or induction. A variety of different magnetically-labeled reagents can be formed by varying either the label component or the binding member component of the reagent. It will be appreciated by one skilled-in-the-art that the choice involves consideration of the analyte to be detected and the desired optimization of the assay technique.

The term "solid phase", as used herein, refers to any material to which analyte, analyte complexes or assay reagents become bound and from which unreacted assay reagents, test sample or test solutions can be separated. The separation of bound and unbound magnetically-labeled reagent may involve the complete removal of the unbound magnetically-labeled reagent from the reaction mixture and/or from that magnetically-labeled reagent which is immobilized on the solid phase. In another embodiment, the separation of bound and unbound magnetically-labeled reagent may involve the sequestering of the unbound magnetically-labeled reagent from that which becomes immobilized upon the solid phase such that the unbound magnetically-labeled reagent remains in the reaction mixture but does not significantly produce a detectable response when the bound magnetically-labeled reagent is placed in the vicinity of a magnetic field. In alternative embodiments, either the unbound or bound magnetically-labeled reagent is observed for a response to a magnetic field. In an alternative embodiment, both the unbound and bound magnetically-labeled reagents are observed for a response to a magnetic field.

The term "ancillary binding member", as used herein, refers to any member of a binding pair which is used in the assay in addition to the binding members of the magnetically-labeled reagent or solid phase. One or more ancillary binding members can be used in an assay. For example, an ancillary binding member can be capable of binding the magnetically-labeled reagent to the analyte of interest, in instances where the analyte itself could not directly attach to the magnetically-labeled reagent. The ancillary specific binding member can be incorporated into the assay device or it can be added to the device as a separate reagent solution.

### Assay Reagents

A wide variety of magnetically attractable materials, suitable for use as magnetic labels in the present invention, are commercially available or the production techniques are well-documented in the art. The required characteristics of the magnetically attractable label are also met by a wide variety of magnetic materials.

In general, the attractive or repulsive force or relative magnetic responsiveness of the material, is the main magnetic property of importance. Magnetically attractable materials include ferromagnetic, ferrimagnetic, paramagnetic and superparamagnetic materials. The term "ferromagnetic" is generally used to describe materials which are attracted to a magnet to a high degree and which typically become permanently magnetized upon exposure to a magnetic field. Ferromagnetic materials may also be reduced in particle size such that each of the particles is a single domain. In this state of subdivision, the ferromagnetic material may be referred to as "superparamagnetic", characterized by the absence of any permanent measurable magnetization. Suitable magnetically repulsed materials include diamagnetic materials including, but not limited to, organic polymers such as polystyrene.

Suitable magnetically attractable materials include metals (e.g., iron, nickel, cobalt, chromium and manganese), lanthanide series elements (e.g., neodymiun, erbium), alloys (e.g., magnetic alloys of aluminum, nickel, cobalt, copper), oxides (e.g., Fe₃O₄, γ-Fe₃O₄, CrO₂, CoO, NiO₂, Mn₂O₃), composite materials (e.g., ferrites) and solid solutions (e.g., magnetite with ferric oxide). Preferred magnetic materials involve magnetite, ferric oxide (Fe₃O₄) and ferrous oxide (Fe₂O₃).

Suitable particle compositions include, but are not limited to, those specific particle types outlined in Table 1.

**TABLE 1**

| **TYPE** | **FORM** | **COMPOSITION** |
|---|---|---|
| Solid particle | | • iron • iron oxide • core of magnetic material, coated with a metal oxide • colloidal magnetic particles containing magnetite or hematite and having a specific gravity of up to 8 and size range of less than 1 to 800 nanometers |
| Layered particles | magnetic material core with a nonmagnetic coating | • a magnetic metal oxide core generally surrounded by a polymeric silane coat • a water-insoluble metal substrate coated with a condensation product of an aminobenzoic acid with an aldehyde, suitable for coupling to a compound having biological affinity |
| | magnetic material core with a nonmagnetic coating | • a core formed of a single particle of magnetically-responsive material with a coating of a water-insoluble cross-linked polymeric material having reactive groups at the surface thereof |
| Layered particles Cont | nonmagnetic core with a magnetic material coating | • an organic polymer particle with a ferrite coating • a sphere of thermoplastic material with a magnetic material coating (on at least a portion of the core surface) • a metal-coated polyaldehyde microsphere • an inner core polymer particle (e.g., polystyrene) with a magnetically responsive metal oxide/polymer coating evenly covering the inner core |
| | nonmagnetic core with a magnetic material layer and a nonmagnetic coating | • an agarose-encapsulated metal-coated polyaldehyde microsphere • a thermoplastic resin bead (e.g., polystyrene, polyvinyl chloride, polyacrylate, nylon, etc.) with from 1-25% by weight of magnetically responsive powder bound on the bead, and a polymer coated thereon having functional groups to bind a biologically active component |
| Composite particles | magnetic material embedded within a nonmagnetic material | • iron-containing magnetic crystals 0.1 µm (<1000 Å) incorporated within a glass and/or crystal structure • a copolymer matrix: 30-99% by weight, of at least one monoethylenic monomer which does not coordinate with a metal complex, 0.5-50% by weight, of at least one crosslinkable polyethylenic monomer which does not coordinate with a metal complex, and 0.5-30% by weight, of at least one nucleophilic monomer which can be coordinated with a metal complex, with encapsulated crystalites of a metal • magnetizable particles of a size less than 0.03 µm (300 Å) encapsulated in an organpolysiloxane matrix • a particulate reaction product of a water-soluble form of iron and a water-soluble polymer having available coordination sites (free electron pair for a coordinate bond with a transition metal atom) • an organic, inorganic or synthetic polymer matrix containing a magnetically attractable material |
| Composite particles Cont. | magnetic material embedded within a nonmagnetic material | • magnetizable particles of a size less than 0.03 µm (300 Å) encapsulated in an organpolysiloxane matrix • a particulate reaction product of a water-soluble form of iron and a water-soluble polymer having available coordination sites (free electron pair for a coordinate bond with a transition metal atom) • an organic, inorganic or synthetic polymer matrix containing a magnetically attractable material • a continuous phase of a water-insoluble polymeric matrix having dispersed (embedded) therein: a magnetically attractable material, and a particulate absorbent material (selected from charcoal, talc, ion exchange resins, Fuller's earth, silicon dioxide, oxides of zirconium or aluminum or titanium, porous glass, zeolites, natural or synthetic polymers, polymerized first or second antibodies or polymerized enzymes, cell surface antigens or receptors in a particulate form, subcellular particles and bacterial cells) • particles made by polymerizing one or more monomers in the presence of magnetically attractable solids to form directly a synthetic water-insoluble polymeric matrix having the solids uniformly embedded therein • particles of cross-linked protein or polypeptide and a magnetically responsive material made by combining: an organic solvent solution of a high MW polymer (e.g., polystyrene), a particulate magnetically responsive material and a polyfunctional cross-linking agent (e.g., polyaldehyde) |
| Matrix particles | magnetic material dispersed within a nonmagnetic material | • hydrophobic vinyl aromatic polymer particles having a mean diameter between 0.03 and 5 µm (microns)and a magnetically-charged material in an amount from 0.5 to 50% by weight with respect to the polymer portion of the particles, the magnetically-charged material being dispersed within the polymer particles • a filler selected from the group consisting of a metal, metal alloy, metal oxide, metal salt, metal sulfide, pigment and metallic chelate compound, and an oleophilic surface layer upon the filler, and a layer of polymer upon the oleophilic-surfaced filler |

Magnetic labels formed as matrix or composite particles may optionally include additional coatings or layers of magnetic or nonmagnetic materials or mixtures thereof. Matrix compositions can be made by any suitable means including the polymerization of the magnetically attractable material with the selected monomer or the swelling of the matrix material with the introduction of the magnetically attractable material into pores within the matrix. The matrix may include organic and inorganic materials such as glass, cellulose, synthetic polymer materials, agarose, etc. Suitable polymer materials include, but are not limited to, polymers of styrene, substituted styrenes, naphthalene derivatives, acrylic and methacrylic acids, acrylamide and methacrylamide, polycarbonate, polyesters, polyamides, polypyrrole, aminoaromatic acids, aldehydes, proteinaceous materials (such as gelatin and albumin), polysaccharides (including starch and dextran) and copolymers of polymeric materials. The polymer may also be used in admixture with an inert filler or may include an absorbent material.

Generally, the magnetic particles used in the present invention are substantially spherical in shape, although other shapes are suitable and may be advantageous in some circumstances. Other possible shapes include plates, rods, bars and irregular shapes.

In general, the diameter of the magnetic label ranges from about 0.01 to about 1.000 µm (microns). The size of the magnetic label is typically under ten microns, but sizing is not critical to the present invention.

In one possible embodiment, the magnetic particles might be selected to have a specific gravity so as to tend to be suspended within the reaction mixture thereby enhancing the reactivity of the binding member. Small magnetic particles with a mean diameter of less than about 0.03 µm (300 Å) can be kept in solution by thermal agitation and do not spontaneously settle. In alternative embodiments, the magnetic particles might be selected to have a specific gravity so as to tend to settle in the reaction mixture thereby enhancing the reactivity of the binding member with the immobilized reagent on the solid phase. Large magnetic particles having a mean diameter greater then 10 µm (microns) can respond to weak magnetic fields. Large or dense labels may be used but may require that the reaction mixture be stirred or agitated during the incubation steps to inhibit settling of the particles. In yet another embodiment, the magnetic particles are selected to remain dispersed in the reaction mixture for a time sufficient to permit the required binding reactions without the need for a stirring or mixing means.

As will be appreciated by those skilled-in-the-art, the composition, shape, size, and density of the magnetically attractable material may vary widely. The desired label attributes are determined empirically and the label is selected depending upon such factors as the analyte of interest and the desired assay protocol.

In forming the magnetically-labeled reagent, the attachment of the binding member to the magnetically attractable material may be achieved by any suitable attachment means including adsorption, covalent bonding, cross-linking (chemically or through binding members) or a combination of such attachment means. Where the binding member is covalently bonded to the magnetically attractable label, the covalent bond may be formed between one component and a chemically active form of the other component, e.g., an active ester such as N-hydroxysuccinimide can be introduced into one component and allowed to react with a free amine on the other component to form a covalent coupling of the two. Other examples include, but are not limited to, the introduction of maleimide onto one component which is then allowed to react with endogenous or introduced sulfhydryl moieties on the other component, or the oxidation of endogenous or introduced carbohydrate groups on one component to form aldehydes which can react with free amines or hydrazides on the other component. Many reagents are commercially available to carry out such modifications and linkages. Suitable homobifunctional and heterobifunctional linker arm reagents are also available to provide such conjugations. Suitable reagents and conjugation techniques are well-known to those skilled-in-the-art. In another embodiment, where the magnetically attractable label includes a polymer coating or matrix, the polymer may be selected so that it contains, or can be provided with, suitable reactive groups such as azide, bromoacetyl, amino, hydroxyl, sulfhydryl, epoxide, carboxylic or other groups to facilitate the attachment of the binding member.

The coupling groups and coupling or linking agents are selected so that the binding activity of the binding member is not substantially modified or destroyed upon attachment to the label. The quantity of binding member which may be attached to the magnetically attractable label depends upon its concentration, the conditions used, and the amount of and nature of the available functional groups on the magnetically attractable label or coupling agent.

The solid phase material and solid phase reagents generally involve nonporous materials including, without limitation, polymers of styrene, substituted styrenes, naphthalene derivatives, acrylic and methacrylic acids, acrylamide and methacrylamide, polycarbonate, polyesters, polyamides, polypyrrole, polypropylene, latex, polytetrafluoroethylene, polyacrylonitrile, polycarbonate, glass or similar materials, aminoaromatic acids, aldehydes, proteinaceous materials (such as gelatin and albumin), polysaccharides (including starch and dextran) and copolymers of polymeric materials. Such materials are typically in the form of particles, beads, tubes, slides, tapes, webbing, plates or wells. Thus, the solid phase may be the "reaction vessel" in which the binding assay takes place, e.g., a microtitre well, or it may be a materials contained within the reaction vessel, e.g., a bead within a test tube.

The solid phase material might also be any suitable chromatographic, bibulous, porous or capillary material. In the present invention, the solid phase material can include, but is not limited to, a fiberglass, cellulose or nylon pad for use in a flow-through assay device having one or more layers containing one or more of the assay reagents; a dipstick for a dip and read assay; a test strip for chromatographic (e.g., paper or glass fiber) or thin layer chromatographic (e.g., nitrocellulose) techniques in which one or all of the reagents are contained in separate zones of a single strip of solid phase material; or an absorbent material well known to those skilled in the art.

As further examples, natural, synthetic or naturally occurring materials that are synthetically modified, can be used as a solid phase material including polysaccharides, e.g., cellulose materials such as paper and cellulose derivatives such as cellulose acetate and nitrocellulose; silica; silicon particles; inorganic materials such as deactivated alumina, or other inorganic finely divided material uniformly dispersed in a porous polymer matrix, with polymers such as vinyl chloride, vinyl chloride polymer with propylene, and vinyl chloride polymer with vinyl acetate; cloth, both naturally occurring (e.g., cotton) and synthetic (e.g., nylon); porous gels such as silica gel, agarose, dextran, and gelatin; polymeric films such as polyacrylates; protein binding membranes; and the like.

The solid phase material should have reasonable strength or strength can be provided by means of a support, and it should not interfere with the production of a detectable signal. In addition, it will be appreciated that the solid phase material is typically nonmagnetic or that its magnetic contribution to the assay is correctable.

The solid phase generally has a binding member immobilized on or in its surface to permit the immobilization of another assay reagent or the analyte. In addition, the immobilized binding member may be selected to directly bind the analyte or to indirectly bind the analyte by means of an ancillary specific binding member.

It is not critical to the present invention that the immobilized reagent be bound directly to the solid phase. The binding member can be attached to another material wherein that material is physically entrapped or retained and immobilized within the solid phase by a physical, chemical or biochemical means. For example, an analyte-specific binding member can be attached to insoluble microparticles which are subsequently retained by a porous material. The means of attaching a reagent to the microparticles encompasses both covalent and non-covalent means. It is generally preferred that the binding member be attached to the microparticles by covalent means. By "retained" is meant that the microparticles, once on the porous material, are not capable of substantial movement to positions elsewhere within the porous material. The microparticles can be selected by one skilled-in-the-art from any suitable type of material including polystyrene, polymethylacrylate, polyacrylamide, polypropylene, latex, polytetrafluoroethylene, polyacrylonitrile, polycarbonate, glass or similar materials.

Solid phase reagents or binding members immobilized on a solid phase include binding members that are attached to the solid phase before, during or after incubation with the test sample and/or other assay reagents. In most embodiments, however, the immobilized reagent is bound or attached to the solid phase prior to contacting the solid phase with the test sample. To simplify the disclosure hereinafter, the solid phase will principally be described as involving a plastic well structure as a reaction vessel containing at least the immobilized binding member necessary for the performance of the desired binding assay.

### Assay Methods

The methods and devices of the present invention may be applied to any suitable assay format involving binding pair members including, but not limited to, those binding members described above. The following examples will typically refer to antibody/antigen binding assays in order to simplify the description.

Various competitive and sandwich assays have been described in the literature whereby a labeled reagent is partitioned between a liquid phase and a solid phase in proportion to the presence of the analyte in the test sample. The present invention is applicable to competitive assay formats. A first binding member is attached to a magnetically attractable label, thereby forming a magnetically-labeled reagent, either before, during or after incubation of the binding member with the test sample. In one possible assay, the analyte in the test sample and a magnetically-labeled analyte analog compete for binding to a binding pair member attached to a solid phase. Alternatively, the binding member attached to the solid phase may be an analyte-analog selected to compete with the analyte for binding to a magnetically-labeled binding pair member. The proportion of magnetically-labeled reagent that becomes bound to the solid phase is inversely related to the amount of analyte in the test sample.

The present invention is also applicable to sandwich assays. A first binding member is attached to a magnetically attractable label either before, during or after incubation of the binding member with the test sample. A second binding member, which is attached to the solid phase to form a solid phase reagent, is selected to directly or indirectly bind the analyte of interest. The magnetically-labeled reagent becomes immobilized upon the solid phase by binding the analyte that has bound the solid phase reagent. Thus, the proportion of magnetically-labeled reagent that becomes bound to the solid phase is directly related to the amount of analyte in the test sample.

Assay formats may optionally involve the use of ancillary binding members to indirectly bind the analyte to the magnetically-labeled reagent or to the solid phase reagent. In addition, the assay protocols may involve incubating the reagents either all together with the test sample or in a specified sequence, and for a time period suitable with analyte binding. After incubation, the unbound magnetically-labeled reagent is separated from the bound magnetically-labeled reagent. The unbound magnetically-labeled reagent may be completely removed from the reaction mixture. Alternatively, the unbound magnetically-labeled reagent may be sequestered from that magnetically-labeled reagent which is immobilized upon the solid phase such that it has no substantial affect upon the magnetically assisted measurement of the immobilized magnetically-labeled reagent.

In the assay methods of the present invention, the response of the magnetically-labeled reagent to the influence of a magnetic field is exploited to provide a qualitative or quantitative measurement of the extent of binding between binding pair members. The presence of an analyte mediates the capture of the magnetically-labeled binding member by the binding member immobilized on the solid phasé. Typically, the magnetically-labeled reagent that is not captured/bound on the solid phase is separated from the solid phase by the application of a magnetic field that is sufficient to move unbound, but not bound, magnetically-labeled reagent away from the solid phase.

The influence of the magnetic field upon the magnetically-labeled reagent may be observed or detected by any suitable means. For example, the change in the apparent weight of the immobilized magnetically-labeled reagent upon application of a magnetic field can be detected by a weighing means. In another embodiment, the change in apparent mass of the immobilized magnetically-labeled reagent upon application of a magnetic field can be detected by a resultant change in frequency of a quartz crystal, etc. In yet another embodiment, the effect of the force exerted upon the immobilized magnetically-labeled reagent may be observed as the physical displacement of the immobilized magnetically-labeled reagent or the displacement of the vessel or material on which the magnetically-labeled reagent is immobilized. An optical sensor means can be used to to assess the magnitude of a change from an initial position to a subsequent position assumed by the immobilized magnetically-labeled reagent, solid phase material or vessel in the field. In a further embodiment, a motion detection means can be used to assess the movement of the immobilized magnetically-labeled reagent upon the application of the magnetic field. For example, in one embodiment a stress sensitive material, such as a piezoelectrical film, may be used to detect motion. In yet another embodiment, stress sensitive materials can be incorporated into the vessel or solid phase material such that upon the application of a magnetic field the change in stress would be detectable. In other embodiments, it may be preferred to detect the unbound magnetically-labeled reagent by a suitable detection means. In other embodiments, it may be preferred to detect both the bound and unbound magnetically-labeled reagent and evaluate the ratio of the partitioning.

The following sequence of steps exemplify one form of sandwich assay using the magnetically assisted detection of a magnetically-labeled reagent:
1) a first binding member, specific for the analyte, is attached to a magnetically attractable label, thereby forming a magnetically-labeled reagent;
2 ) a second binding member, specific for a second binding site on the analyte, is attached to a solid phase, thereby forming an immobilized binding member or solid phase reagent;
3 ) the test sample is contacted with the immobilized binding member whereby analyte becomes bound to the solid phase by means of the analyte-specific binding member;
4 ) the solid phase is contacted with the magnetically-labeled reagent whereby the magnetically-labeled reagent becomes immobilized upon the solid phase by binding the captured analyte (the proportion of magnetically-labeled reagent that becomes bound to the solid phase is directly related to the amount of analyte in the test sample);
5 ) the unbound magnetically-labeled reagent is removed from the solid phase;
6 ) the solid phase is placed on a weighing pan of a microbalance, and the balance is zeroed;
7 ) the solid phase is exposed to a magnetic field such that a magnetic force is exerted on the magnetically-labeled reagent immobilized upon the solid phase, and the influence of this force upon the captured magnetic reagent is manifested as a deviation of the balance readout from zero; and
8 ) the degree of the measurable balance deviation provides a direct measure of the quantity of the captured magnetic microparticles on the solid phase.
The immobilized non-magnetic reagents and test sample components have no influence upon this measurement

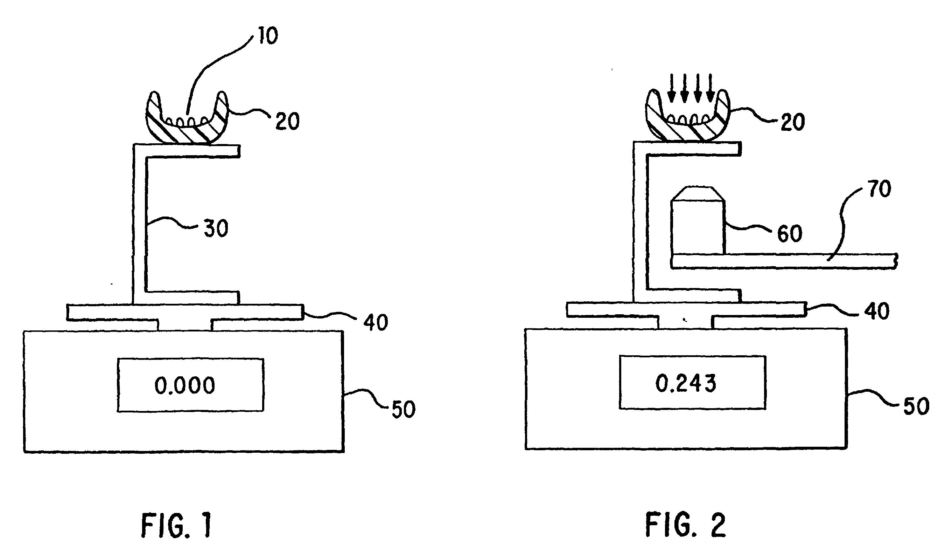

Figure 1 depicts a schematic view of the magnetically assisted measurement of the binding of a magnetically-labeled reagent to a solid phase. The solid phase, e.g., a well (20) contains the immobilized magnetically-labeled reagent (10) following the selected assay format. The solid phase is set upon or affixed to a support means (30). The support means rests upon a balance means (50). On a typical microbalance, the balance has a pan (40) which will receive the support. Once the balance receives the support, or once the support receives the solid phase, the balance can be set to equilibrium (zeroed).

In Figure 2, a magnet (60) is inserted into the vicinity of the well, whereby the magnetic field exerts a force upon the magnetically-labeled reagent immobilized on the solid phase. The force exerted upon the magnetically-labeled reagent is manifested as an apparent change in the weight of the solid phase which is registered on the scale of the balance means (50). Generally, the magnet is affixed to an arm means (70) which allows delicate adjustments of the movement of the magnet towards and away from the solid phase.

The magnetic field may be provided by means of a permanent magnetic or an electromagnet and may be applied intermittently or continuously. An electromagnetic may be used so that the magnetic field can be turned off and on rather than requiring the movement of the magnet or of the solid phase. An electromagnet can be computer controlled, thereby providing for fine adjustments to magnetic field strength. Furthermore, an electromagnet can be used to generate an alternating magnetic field which can have the further advantage of causing the mixing of the magnetically-labeled reagent in the reaction mixture if such mixing is desired.

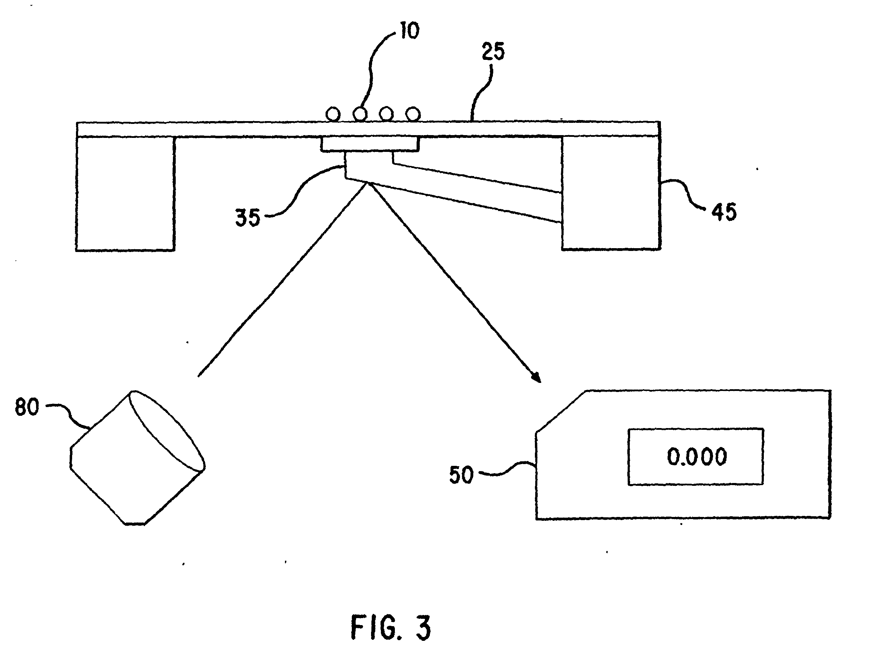

Figure 3 depicts a schematic view of an alternate means for the measurement of the binding of a magnetically-labeled reagent to the solid phase. The solid phase, e.g., a strip of bendable material (25) contains the immobilized magnetically-labeled reagent (10) following the selected binding reaction. The solid phase is supported on a foundation means (45). The detection means includes a cantilever beam (35) which contacts the solid phase to detect any movement of the solid phase. Detection is accomplished by means of a laser light source (80) and an optical sensor means (50). Coherent light from the laser is reflected from the cantilever beam onto the optical sensor. Any deviation in the position of the cantilever beam results in a shift of position or deflection of the reflected light striking the optical sensor, thereby causing a change in its output. The greater the distance traveled by the light, the greater the sensitivity of such a measurement means.

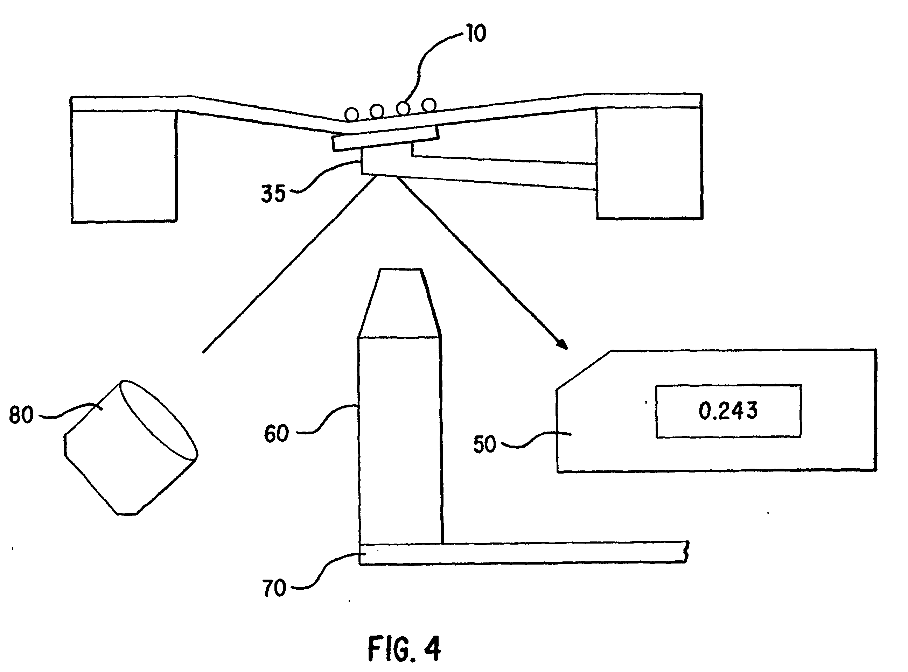

In Figure 4, a magnet (60) is positioned in proximity to the bendable material, whereby the magnetic field exerts a force upon the magnetically-labeled reagent immobilized on the solid phase. The force exerted upon the solid phase will cause a displacement of the material or a distortion in the shape of the material. The degree of displacement or distortion from the original position of the solid phase can be observed by the detection means.

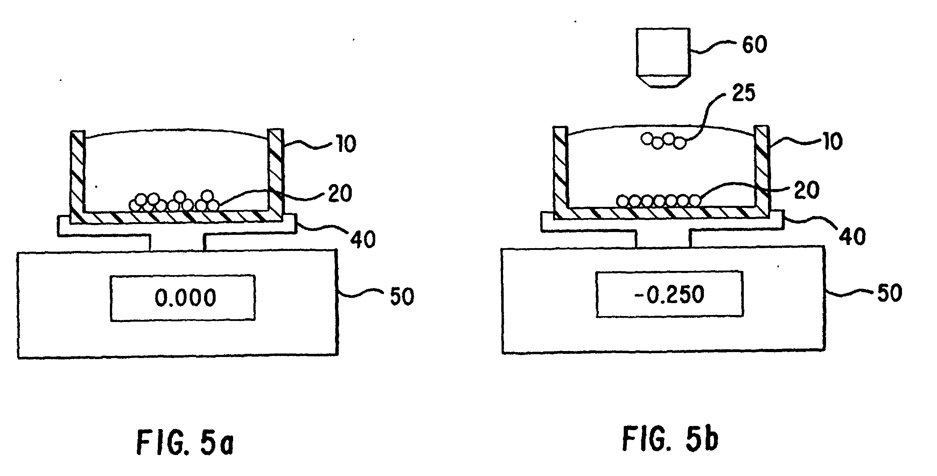

Figure 5(a) depicts schematic views of yet another means for the determination of the binding reaction by detecting the force exerted upon the unbound magnetically-labeled reagent. The solid phase, e.g., a well (10), contains magnetically-labeled reagent (20) at least a portion of which is immobilized in the well following the selected assay format. The solid phase is set upon or affixed to a balance means (50). In this example, the balance has a pan (40) which will receive the well. Once the balance receives the well, the balance can be set to equilibrium (zeroed). In Figure 5(b), a magnet (60) is inserted into the vicinity of the surface of the well contents, whereby the magnetic field exerts a force upon the magnetically-labeled reagent. Under the influence of this force the unbound magnetically-labeled reagent (25) migrates to the air-liquid interface where the magnetic attraction is more intense due to the closer proximity of the magnet. The bound magnetically-labeled reagent resists movement under this level of magnetic field intensity and remains bound through the analyte to the well bottom. The magnetically-labeled reagent at the air-liquid interface strains upward against the surface tension of the liquid surface, causing a change in the apparent weight of the solid phase which is registered as a change of the readout on the scale of the balance means (50). As the magnet is moved closer to the well, the greater the amount of free reagent at the air-liquid interface and the increased intensity of the magnetic field results in a greater change in the apparent weight of the solid phase. As the magnetic field intensity increases, the weaker association of non-specifically bound magnetically-labeled reagent with the well bottom will be overcome, thereby separating it from the specifically bound magnetically-labeled reagent.

The method illustrated in Figure 5 can also be used to determine the strength of the association between binding members. As the magnetic is moved closer to the well, the increasing intensity of the magnetic attractive force at the well bottom begins to pull specifically bound magnetically-labeled reagent from the well bottom to the air-liquid interface where it makes a greater contribution to the aggregate upward force on the well. A method for determining the strength of the association between binding members is described in greater detail hereinafter.

From these examples, it will be appreciated by those skilled-in-the-art that the present invention is not dependent upon the means used to detect the effect of the magnetic field upon the magnetically-labeled reagent. A wide variety of instruments can be used to detect mass changes, position changes, movement of the magnetically-labeled reagent, movement of a diamagnetic-labeled reagent, increased weight, decreased weight, force changes, magnetic susceptibility and induction, all of which result from the interaction between a magnetic field and the magnetically-labeled reagent.

Because the present invention involves the assessment of the magnetic responsiveness of the label, the various detection methods are readily adaptable to an automated operation. As used herein the term "automated" is not meant to exclude the possibility that some assay operations may be carried out manually.

A magnetic means may also be used to separate the unbound or free magnetically-labeled reagent from the bound or immobilized reagent. For example, the unbound magnetically-labeled reagent may be removed from the reaction mixture by inserting a magnetic probe into the reaction mixture and then removing the probe with any unbound magnetically-labeled reagent that is attracted to that probe. In another embodiment, the unbound magnetically-labeled reagent may be pulled from the reaction mixture by placing a magnet outside of the reaction vessel and moving the magnet along the vessel bottom and/or wall, thereby pulling the unbound magnetically-labeled reagent from the reaction mixture or away from that reagent immobilized on the solid phase. The unbound magnetically-labeled reagent may be completely removed from the reaction mixture and the solid phase or it may be moved away from the immobilized magnetically-labeled reagent such that the free reagent does not influence the detection of the bound magnetically-labeled reagent. In yet another embodiment, a magnetic means may be brought into proximity with the surface of the reaction mixture such that unbound magnetically-labeled reagent is sequestered at the air/liquid interface of the reaction mixture, thereby separating unbound magnetically-labeled reagent from the immobilized reagent. In a further embodiment, the unbound reagent may be moved away from the immobilized magnetically-labeled reagent and retained in a suitable manner such that the unbound reagent is retarded from moving back to the area of the solid phase on which the bound reagent is immobilized.

### EXAMPLES

The following examples describe assays which were performed in accordance with the present invention as well as a device for performing the assays.

### Example 1

### Magnetically Assisted Magnetic Label Measurement

A plastic support was inserted into the pan receptacle of a electronic balance (Mettler AE 163; Mettler Instrument Corporation, Heightson, NJ). The support had a hole in the top cross piece which would accommodate a single microtiter well (Nunc snap-apart, eight well module strips; Nunc Incorporated, Naperville, IL) so that the bottom of the well would extend below the bottom of the cross piece. A neodymium-iron-boron fixed magnet (Racoma Incorporated, Boonton, NJ) was placed on the end of a plastic bar attached to the mechanical stage of a microscope so that movement of the mechanical stage allowed the accurate and reproducible positioning of the magnet below the bottom of the suspended microtiter well.

The paramagnetic particles were polystyrene/vinyl/COOH/magnetite particles (Bang's Laboratories, Incorporated, Carmel, IN) supplied as a 10% (w/v) suspension. This suspension was diluted ten-fold with water to a concentration of 10 milligrams of particles per milliliter. Aliquots ranging from 5-40 microliters (50-400 µg of particles) were pipetted into the microtiter well which was inserted into the support. The particle suspension was further diluted ten-fold to obtain another set of aliquots containing 5-40 micrograms of particles to assess the response from smaller quantities of particles. A measured quantity of magnetic particles was placed in the microtiter well, and the balance was zeroed with the magnet withdrawn.

In order to measure the force generated upon approach of the magnet to the magnetic particles in the well, the magnet was positioned approximately two millimeters below the well bottom and was moved toward the well bottom in a series of small incremental moves. The balance readout was noted after each movement, and the last reading obtained before the magnet touched the well bottom was recorded. Using this procedure, an average enhancement factor of over 700 was observed, and the effect appeared substantially linear down to at least 20 micrograms of magnetic particles as illustrated by the data presented in Table 2.

**TABLE 2**

| Weight of magnetically-labeled reagent in a well (micrograms) | Force on the reagent due to magnetic field (micrograms) | Ratio of Weight/Force |
|---|---|---|
| 5 | 4,500 | 900 |
| 11 | 3,300 | 300 |
| 16 | 11,000 | 687 |
| 21 | 13,500 | 642 |
| 27 | 20,000 | 740 |
| 32 | 25,000 | 781 |
| 38 | 25,000 | 658 |
| 43 | 33,000 | 767 |
| 54 | 40,000 | 741 |
| 110 | 97,000 | 880 |
| 160 | 150,000 | 937 |
| 210 | 163,000 | 776 |
| 270 | 130,000 | 481 |
| 320 | 310,000 | 968 |
| 380 | 332,000 | 874 |
| 430 | 275,000 | 640 |

### Example 2

### Magnetically Assisted Avidin-Biotin Binding Assay

The following reagents and samples were used in a binding assay:

The magnetically-labeled reagent was a streptavidin-coated paramagnetic microparticle (Advanced Magnetics, Cambridge, MA; average one micron in diameter, with 5x10⁸ particles per mg, supplied as a 5 mg/ml suspension). The binding capacity of the particles was 3.2 micrograms of biotin per milliliter of suspension. Test samples contained various concentrations of biotin in a phosphate buffered saline solution.

The solid phase involved snap-apart polystyrene microtitre wells (Nunc 8-well microwell module strips) which had been coated with biotinylated bovine serum albumin (biotin-BSA; Sigma Chemical Company, St. Louis, MO; 8.9 moles of biotin per mole of BSA). The biotin-BSA was dissolved in phosphate buffered saline (PBS), pH 7.2 to a concentration of 50 micrograms/milliliter, and aliquots (100 µl) were pipetted into each well. Following incubation for one hour at 37° C, the solution was removed from the wells and replaced with 400 microliters of 1% BSA (unbiotinylated) in PBS as an overcoat. Incubation was continued at 37° C for an additional 45 minutes. The wells were then emptied and washed with PBS using a wash bottle. The overall result of this procedure was to immobilize biotin molecules to the bottom of the microtiter wells (as biotin-BSA) and to inactivate the wells to further non-specific binding of protein by overcoating with unbiotinylated BSA.

The magnetically-labeled reagent was first combined with the test sample, thereby forming a reaction mixture which was incubated for one hour at 37° C. An aliquot (80 µl) of each reaction mixture was transferred to the solid phase, where it was further incubated for one hour at 37° C to effect avidin-biotin binding. The unbound magnetically-labeled reagent was removed from the reaction mixture by means of magnetic attraction.

Once combined, the free biotin from the test sample proceeded to bind to the available biotin-binding sites on the avidin moieties of the avidin-coated magnetic particles, thereby inhibiting the subsequent capture of the magnetically-labeled reagent by the immobilized biotin on the well bottom. The degree of inhibition depended on the concentration of the free biotin in the test sample. Thus, the amount of magnetically-labeled reagent that was bound by the solid phase was inversely proportional to the amount of biotin in the test sample.

The magnetic responsiveness of the magnetically-labeled reagent bound to the bottom of each well was determined using an apparatus substantially as described in Example 1, above. The weight change due to the magnetic responsiveness of the immobilized magnetically-labeled reagent in each well was recorded as a function of the quantity of free biotin from the test sample present during incubation.

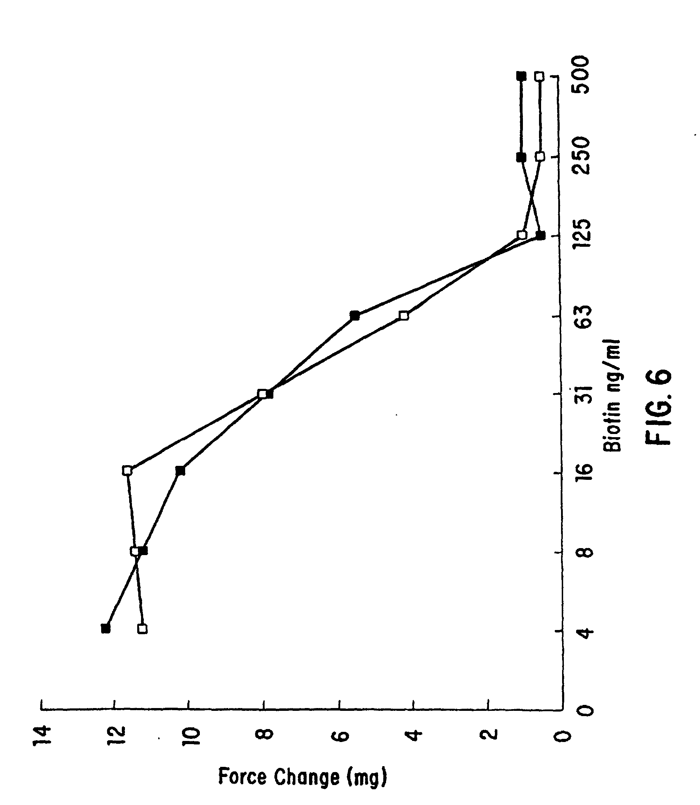

Figure 6 depicts the assay results. The balance means detected a decreasing force change, from 12 milligrams to zero milligrams, as the free biotin concentration in the test sample was increased from 0 nanograms/milliliter to 125 nanograms/milliliter (80 µl assayed). Thus, as the amount of free biotin was increased in a test sample, the amount of magnetically-labeled reagent which bound to the solid phase proportionately decreased, and there was a corresponding decrease in the apparent weight change of that bound reagent upon the application of a magnetic field.

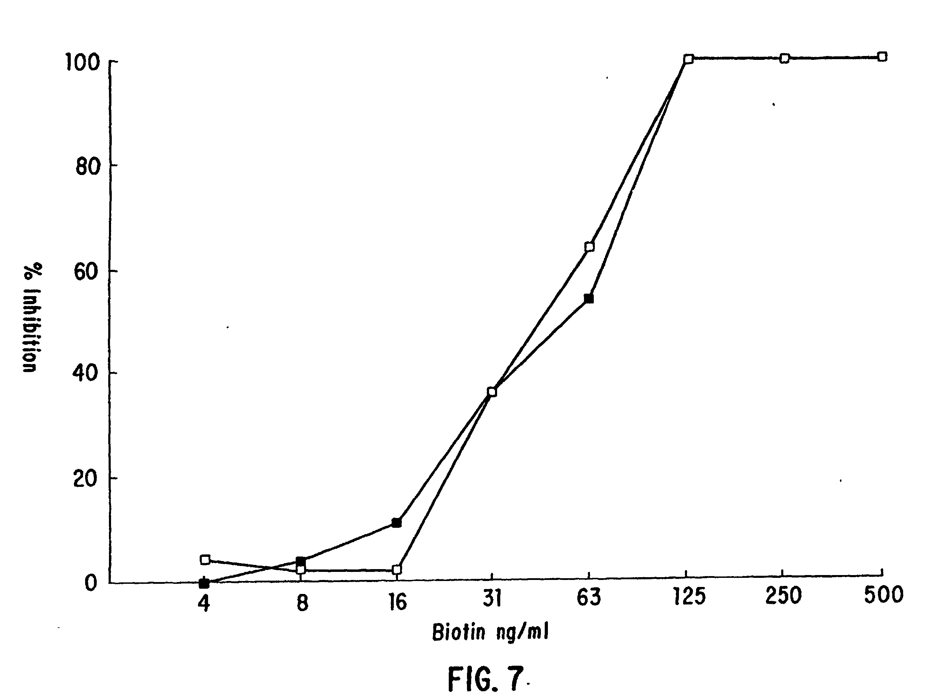

Figure 7 illustrates the assay results plotted as a percent inhibition of the magnetically enhanced weight of the captured magnetically-labeled reagent resulting from the presence of free biotin in the test sample. Fifty percent inhibition was observed at a free biotin concentration of 40 nanograms/milliliter. From these results, it was determined that the assay configuration provided an assay for free biotin in the test sample with a sensitivity in the femtomolar range.

### Example 3

### Balance Means and Magnetically Assisted Measurements

To further explore the potential of the magnetically assisted magnetic label assay concept, a Cahn Model D-200 electronic microbalance (Cahn Instruments Incorporated, Cerritos, CA) was used. This balance consists of a balance beam connected to the rotor of an electric motor. A movement of the beam, as the result of the presence of a weight in one of the hanging pans, is sensed by an optical positioning device, and a current is sent to the motor sufficient to return the beam to its original position. The magnitude of this current is converted by the electronic circuitry of the balance into a weight readout.

An apparatus for precisely positioning a fixed magnet was designed and assembled from three precision positioning tables (Daedal Division of Parker Hannifin Corporation, Harrison City, PA). Two of the tables were micrometer adjusted and used to position the magnet in the horizontal X and Y directions. The third table, mounted vertically, was driven by a microstepper motor to control the movement of the magnet in the vertical, or Z direction (Compumoter Division of Parker Hannifin Corporation, Rohnert Park, CA). A fixed magnet (Racoma 35; Recoma, Inc., Boonton, NJ) was attached to the Z table by a bracket which positioned the magnet inside an enclosure that surrounded the balance pans to shield the pans from air currents. The motor movement was controlled by microprocessor circuitry interfaced with a computer. Acceleration, velocity and distance of movement, as well as final position were programmed into the computer such that complex repetitive movements could be executed automatically. A movement of one inch comprised 100,000 microsteps, and controlled movements of one microstep were possible.

The relationship of the magnetic enhancement factor to the quantity of magnetically-labeled reagent was confirmed using the Cahn balance. A ten microliter aliquot of a suspension containing paramagnetic particles (100 µg/ml; as described in Example 1, above) was pipetted onto the balance pan, the balance was equilibrated, the magnet was brought into proximity of the pan and the change in readout was noted. Additional ten microliter aliquots were then added, and the process was repeated until a total of 100 microliters had been added. Table 3 illustrates the relationship between the quantity of magnetically-labeled reagent in the well and the force exerted upon the reagent (measured as an increase in weight) by the magnetic field: as the quantity of magnetic reagent increased, the balance deviation due to the magnet movement increased linearly.

**TABLE 3**

| Balance readout (milligrams) | | | | |
|---|---|---|---|---|
| Quantity of paramagnetic microparticles in well (micrograms) | magnet away | magnet close | Difference | Ratio |
| 1 | 8.05 | 8.74 | 0.69 | 690 |
| 2 | 17.68 | 18.70 | 1.02 | 510 |
| 3 | 27.19 | 28.65 | 1.46 | 487 |
| 4 | 36.78 | 38.91 | 2.13 | 533 |
| 5 | 46.18 | 49.09 | 2.91 | 582 |
| 6 | 55.06 | 58.80 | 3.74 | 623 |
| 7 | 63.80 | 68.29 | 4.49 | 641 |
| 8 | 72.39 | 77.70 | 5.31 | 664 |
| 9 | 80.63 | 86.86 | 6.23 | 692 |
| 10 | 88.76 | 95.88 | 7.12 | 712 |

### Example 4

### Magnetically Assisted Antibody Assay

A magnetically assisted inhibition immunoassay was demonstrated using the following reagents:

The magnetically-labeled reagent involved paramagnetic particles (Advanced Magnetics) coated with antibody directed against mouse IgG (heavy and light chain) as a one milligram/milliliter suspension (5x10⁸ particles/ml).

The solid phase involved the wells of a microtitre plate which had been coated with mouse IgG (100 microliters of a 50 µg/ml solution, in a 1% carbonate buffer, pH 8.6). The solid phase was then overcoated with 1% BSA in PBS.

Test samples (80 microliters) contained various concentrations of free mouse IgG in a PBS buffered solution.

The magnetically-labeled reagent was incubated with the antibody immobilized on the solid phase in the absence of free mouse IgG. The magnetically-labeled reagent bound to the solid phase and resisted removal upon application of a magnetic field. The binding of the magnetically-labeled reagent was shown to be specific for the immobilized mouse antibody, because the same magnetically-labeled reagent was removed by the application of the same magnetic field when incubated with a solid phase which had been coated with BSA alone.

Upon the incubation of magnetically-labeled reagent with the free mouse antibody the subsequent binding of the magnetically-labeled anti-mouse IgG to the mouse IgG-coated on the well bottom was inhibited. The quantity of magnetically-labeled reagent remaining bound to the solid phase, after the magnetic separation of unbound magnetically-labeled reagent, was measured by placing the well on a balance pan, zeroing the balance, and then moving the magnet into position.

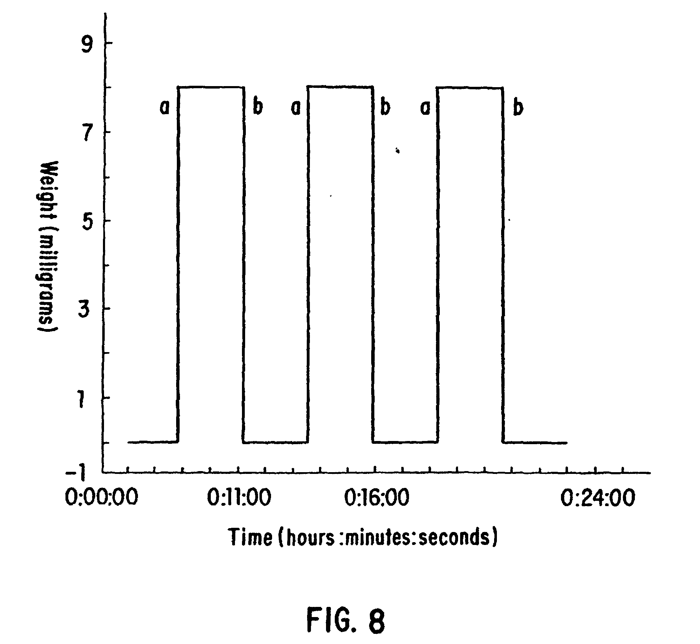

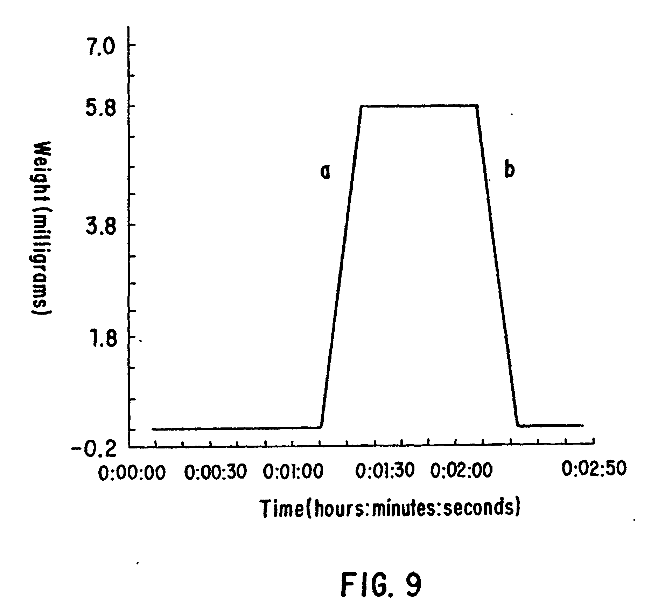

Figure 8 illustrates the effect of repeatedly moving the magnet first towards and then away from the proximity of the bottom of the well in which there had been no free mouse IgG during incubation, i.e., no inhibition of magnetically-labeled reagent binding to the immobilized antibody in the well bottom; wherein (a) shows the magnetic enhancement of weight upon the approach of the magnetic field and (b) shows the return to the zero point upon the removal of the magnetic field. Figure 9 depicts the record of a single cycle of the application and withdrawal of the magnetic field under these conditions, which produced an apparent weight change of 5.8 milligrams.

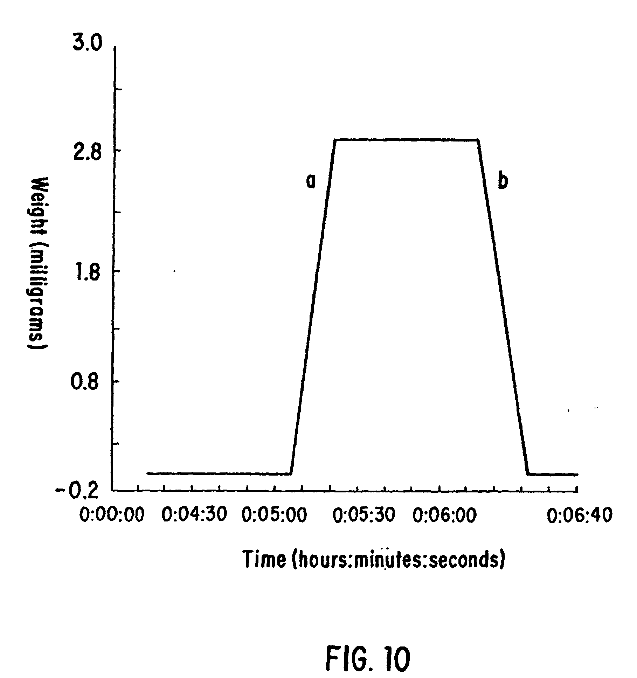

The presence of free mouse IgG (at a concentration of 2.5 µg/ml), during the incubation, caused a decrease in the observed effect of the magnetic field. As depicted in Figure 10, incubation of the magnetically-labeled reagent with free mouse IgG at a concentration of 2.5 micrograms/milliliter resulted in a change of apparent weight from zero to 2.8 milligrams (note the change in vertical axis units from Figure 9). The effect of the presence of various concentrations of free mouse IgG during the incubation was determined as percent inhibition of the value obtained in the absence of free mouse IgG.

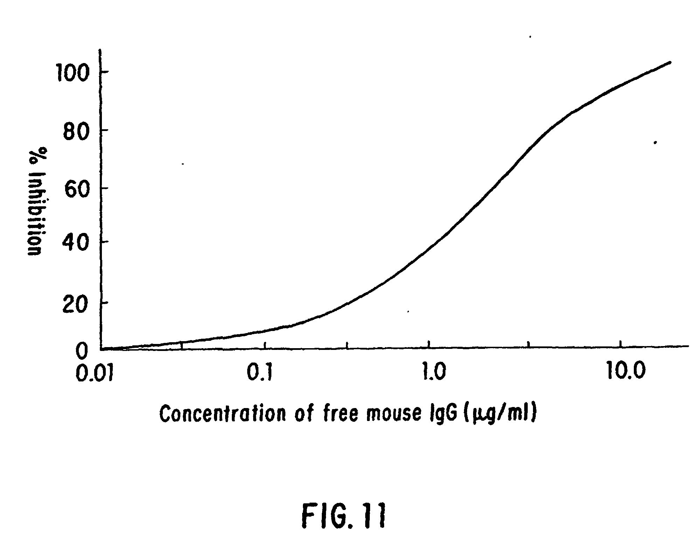

Figure 11 depicts the results which were plotted as a function of free mouse IgG concentration versus per cent inhibition of weight change. The data illustrate a classical inhibition curve with 50 % inhibition resulting from the presence of free mouse IgG at a concentration of one microgram/milliliter.

### Example 5

### Magnetically Assisted Binding Affinity Measurements

The following experiment was performed to measure the binding affinity between a given pair of binding members. The method involved the use of microtiter wells, which had been cut to provide a reduced well wall height of approximately five millimeters, and a magnetic means which approached the wells from above.

The strength of the association between the captured magnetically-labeled reagent and the solid phase was measured. Mouse IgG was immobilized in the well, a suspension of anti-mouse IgG antibody coated magnetic particles was placed in the well and the reaction mixture was incubated to allow binding to take place.

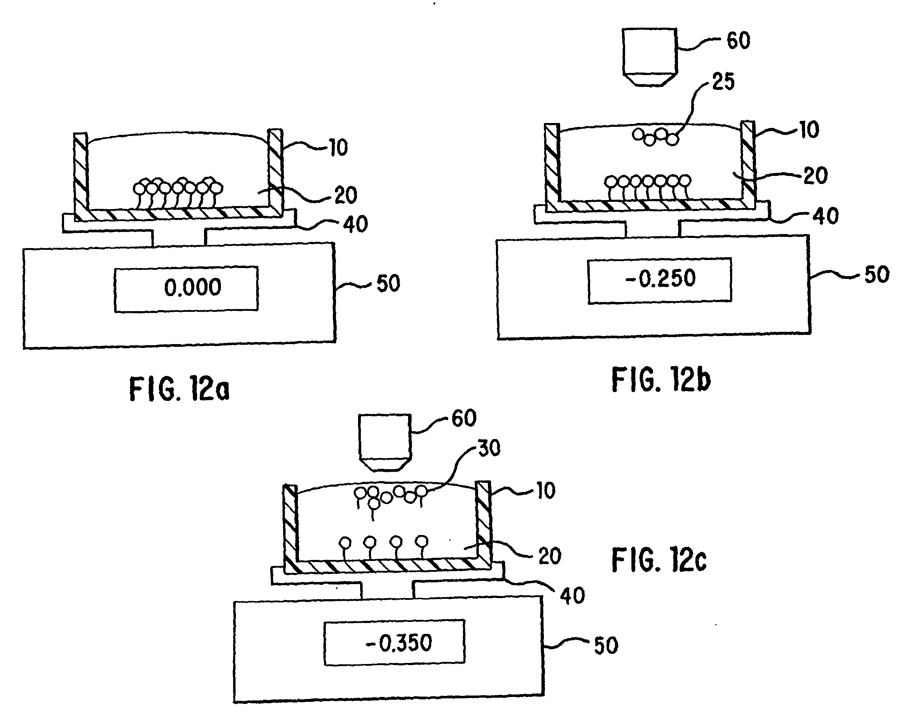

A magnetic field was moved in discrete steps into proximity with the top of the well, thereby causing a controlled series of increases in the upward attractive force exerted on the magnetically-labeled antibody in the well. Figure 12 illustrates the procedure, wherein 12(a) depicts the magnetically-labeled reagent some of which is immobilized on the solid phase as a result of a binding reaction and the balance readout prior to the approach of a magnetic field to the solid phase. The initial movement (50,000 microsteps) of the magnetic field toward the surface of the suspension caused that magnetically-labeled antibody which was not bound to the immobilized antibody to migrate to the air-liquid interface of the suspension. As the magnet was moved closer to the surface (in 5,000 microstep movements) there was a corresponding increase in the attractive force upon the free particles resulting in an observable decrease in the weight of the vessel with each discrete movement, as depicted in 12(b). The free particles collected at the interface exerted an upward force against the surface tension of the liquid surface, thereby causing an observable decrease in the apparent weight of the well. The decrease in weight was determined by a balance means substantially in accordance with the method described in Example 1, above. The magnetically-labeled antibody which was bound to the immobilized antibody on the well bottom also exerted an upward force in the magnetic field. The force exerted by the bound reagent, however, was less than that exerted by the free reagent at the surface due to the greater distance of the bound reagent from the magnet. As the magnet approached the top of the well, thereby causing the upward force on the magnetically-labeled antibody to increase, the magnetically-labeled antibody that was bound to the immobilized binding member on the well bottom began to dissociate from the well bottom and migrate to the liquid surface, as depicted in 12(c).

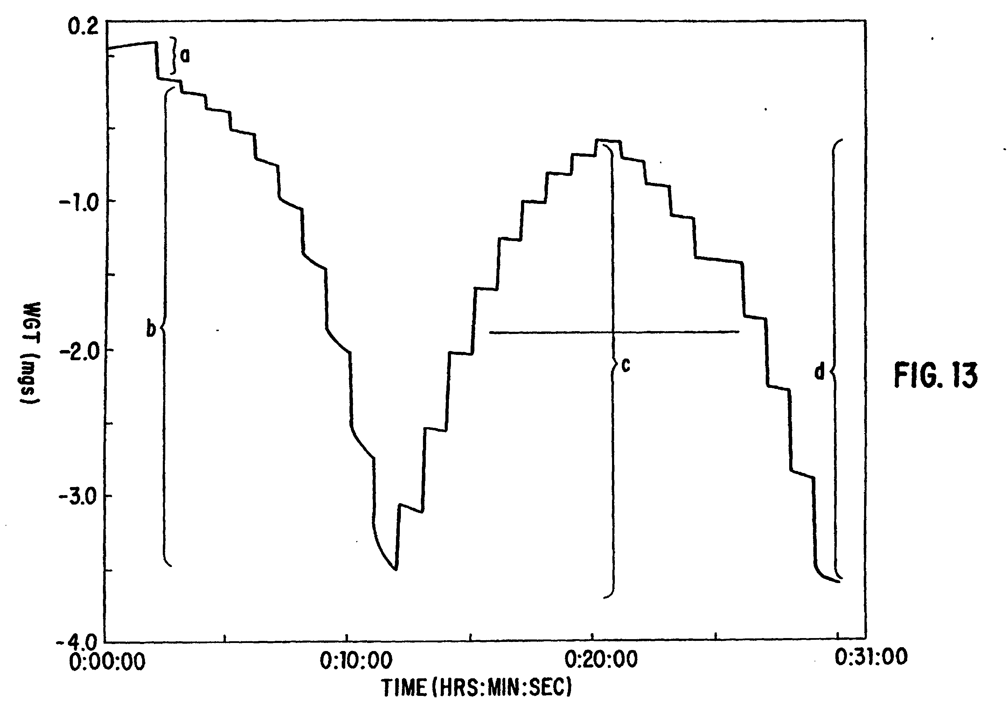

Figure 13 illustrates the measurement of the association force of the magnetically-labeled reagent and the solid phase. During the initial 50,000 microstep movement 13(a) of the magnet toward the top of the well, the attractive magnetic field was relatively weak, and the decreases in apparent weight resulted from the increased upwards force exerted upon the free magnetically-labeled antibody which was collected at the air-liquid surface. As the magnet was moved closer to the surface (in 5,000 microstep movements) there was a corresponding increase in the attractive force upon the free particles resulting in an observable decrease in the weight of the vessel with each discrete movement, as depicted in 13(b). There was no observed weight change when the magnet was stopped between movements, indicating that no change in particle position was occurring between the changes in magnetic field intensity.

As the magnet approached the top of the well, the magnetically-labeled antibody that was bound to the immobilized binding member on the well bottom began to dissociate and migrate to the liquid surface. As the dissociated particles reached the liquid surface, they were in a region of a greater attractive magnetic force, and therefore, these particles exerted a greater upward force on the well. This forced dissociation of the magnetically-labeled antibody from the well bottom, and the subsequent migration to the liquid surface, was manifested as a gradual decrease in well weight between movements of the magnet. The change in apparent weight was seen as a deviation of the weight trace from the horizontal between movements 13(b) of the magnet. As the magnet was withdrawn in a series of discrete movements 13(c) from the proximity of the the well, the apparent weight changes between movements reverted to zero.

When the magnet was again advanced toward the same well 13(d) , there were no apparent weight changes between the movements of the magnet. These occurrences demonstrated that all of the magnetically-labeled reagent which would dissociate from the well bottom under a given level of magnetic force had already dissociated during the first approach of the magnetic field. Thus, any further changes in apparent weight were due to changes in the magnetic field force as the magnet moved, with no contribution due to the further disassociation of magnetically-labeled reagent from the well bottom.

The results demonstrated that the association constants between binding members can be quantitatively determined by means of magnetically assisted magnetically-labeled reagent measurements. The attractive magnetic field intensity required to overcome the association of the binding members is a direct measure of the association constant between the binding members.

### Example 6

### Magnetically Assisted Binding Measurements of Unbound Reagent

A BSA-coated vessel was incubated with a suspension of anti-mouse IgG-coated magnetic particles. The magnetic response measurements (performed substantially in accordance with the method describe in Example 5, above) revealed a decrease of three milligrams in weight when the magnetic means approached the top of the vessel. A vessel containing the same quantity of anti-mouse IgG-coated magnetic particles, but which also contained immobilized mouse IgG (overcoated with BSA), exhibited less than a one milligram weight change, thereby indicating that enough magnetically-labeled reagent had been captured by the immobilized antibody on the well bottom to decrease by two-thirds the magnetic response measurement due to the unbound magnetic particles which had migrated to the liquid surface.

The addition of free mouse IgG, during the incubation of the magnetically-labeled reagent and the solid phase, was found to inhibit the binding of the magnetically-labeled reagent to the immobilized mouse IgG on the well bottom, thereby allowing the unbound magnetically-labeled reagent to migrate to the liquid surface under the influence of the magnetic field and display a greater magnetic responsiveness. In this way, inhibition immunoassays similar to that shown in Figure 11 could be monitored by apparent weight change caused by magnetic particle levitation, i.e., detection of unbound magnetically-labeled reagent. Because magnetic attraction falls off rapidly with distance (as previously shown in Figure 7), the movement of the free magnetically-labeled reagent nearer to the magnet greatly enhances its influence relative to that of the bound magnetically-labeled reagent. This permits a determination of the relative degree of magnetically-labeled reagent binding without requiring the removal of the free magnetically-labeled reagent from the well.

### Example 7

### Separation Means

A solid phase vessel was constructed to include two wells or depressions, of different sizes, which were connected by a narrow channel. The wells were filled with a suspension of magnetic particles. Under the influence of a magnetic field applied from above the larger well, the magnetic particles formed a dot on the surface of the liquid within the larger well. By moving the magnet from above the larger well to above the smaller well (while maintaining the magnetic field), the particles were made to migrate through the channel along the liquid surface until the particles were suspended over the smaller well. The removal of the magnetic field then caused the magnetic particles to fall to the bottom of the smaller well. This illustrated only one of many possible methods by which free magnetically-labeled reagent can be physically separated from magnetically-labeled reagent bound to an immobilized binding member on a solid phase by the influence of a magnetic field.

### Example 8

### Magnetically Assisted Binding Measurements in a Two Particle Assay

An alternative assay method may involve the use of a particulate solid phase. The magnetically-labeled reagent, may involve a binding member conjugated to a magnetic label having an average diameter of 0.05 microns or less. The magnetically-labeled reagent may be mixed with an amount of larger nonmagnetic particles (e.g., polystyrene microparticles, diameter of 5.0 microns) to form a reaction mixture. An immobilized binding pair member on the surface of the nonmagnetic particles causes the magnetically-labeled reagent to bind to the larger particles in the presence of the analyte.

Following a binding reaction, the application of a magnetic field causes the unbound magnetically-labeled reagent to rapidly migrate toward the magnetic means. The magnetically-labeled reagent that is bound to the more massive nonmagnetic particles migrates at a much slower rate in the magnetic field, thereby providing for the discrimination between bound and free magnetically-labeled reagent. After the separation of the unbound magnetically-labeled reagent, that magnetically-labeled reagent that is bound to the non-paramagnetic particles is subjected to analysis using the magnetically assisted magnetically-labeled measurement methods described above.

It will be appreciated by one skilled in the art that many of the concepts of the present invention are equally applicable to other analytes, binding pair members, assay formats and magnetically attractable materials. The specific embodiments described are intended as examples rather than as limitations.

## Claims

1. A method for measuring the association between a first and a second binding member, comprising:
a) reacting said first and said second binding members thereby forming a binding pair complex, wherein said first binding member is attached to a solid phase and said second binding member is attached to a magnetically attractable label thereby forming a magnetically-labeled reagent;
b ) applying and adjusting a magnetic field such that a force acts on said magnetically-labeled reagent so as to dissociate said magnetically-labeled reagent from said solid phase;
c) monitoring the dissociation of said magnetically-labeled reagent from said solid phase; and
d) determining said force necessary to dissociate said binding pair complex, the magnitude of said force being dependent upon the association between said first and said second binding members.

2. The method as recited in claim 1 wherein said dissociation of said magnetically-labeled reagent from said solid phase is monitored by a visual means.

3. The method as recited in claim 1 wherein said dissociation of said magnetically-labeled reagent from said solid phase is monitored by determining the resultant change in force exerted upon said solid phase.

4. A method for differentiating a specific association from a non-specific association between a solid phase and a magnetically attractable label, wherein said specific association is due to a specific binding between a first binding member attached to said solid phase and a second binding member attached to a magnetically attractable label in the form of a magnetically-labeled reagent, said method comprising the steps of:
a) reacting said first binding member attached to said solid phase and said magnetically-labeled reagent thereby forming a binding pair complex;
b ) applying and adjusting a magnetic field such that a force acts on said magnetically-labeled reagent so as to dissociate said magnetically-labeled reagent from said solid phase;
c) monitoring dissociation of said magnetically-labeled reagent from said solid phase;
d) reacting steps a), b) and c) in the presence of a free binding pair member not attached to said solid phase whereby said free binding pair member inhibits the formation of said binding pair complex; and
e) monitoring the difference in forces necessary to dissociate said magnetically-labeled reagent from said solid phase in the absence and the presence of said free binding pair member, said difference in forces being dependent upon said specific association.

5. The method according to claim 4 wherein said free binding pair member is the same as said first binding member.

6. The method according to claim 4 wherein said free binding pair member is the same as said second binding member.

## Patentansprüche

1. Ein Verfahren zur Messung der Assoziation zwischen einem ersten und einem zweiten Bindeglied, das folgendes umfaßt:
a) Reagieren des ersten und des zweiten Bindeglieds, wobei sich ein Bindungspaarkomplex bildet, wobei das erste Bindungsglied an eine feste Phase angeheftet ist und das zweite Bindungsglied an einen magnetisch anziehbaren Marker angeheftet ist, wobei sich ein magnetisch-markiertes Reagenz bildet;
b) Anlegen und Abstimmen eines magnetischen Feldes, so daß eine Kraft auf das magnetisch-markierte Reagenz wirkt, um das magnetisch-markierte Reagenz von der festen Phase zu dissoziieren;
c) Überwachen der Dissoziation des magnetisch-markierten Reagenzes von der festen Phase; und
d) Bestimmen der Kraft, die notwendig ist, um den Bindungspaarkomplex zu dissoziieren, wobei die Größe der Kraft abhängig ist von der Assoziation zwischen den ersten und den zweiten Bindegliedern.

2. Das Verfahren gemäß Anspruch 1, worin die Dissoziation des magnetisch-markierten Reagenzes von der festen Phase durch ein visuelles Mittel überwacht wird.

3. Das Verfahren gemäß Anspruch 1, worin die Dissoziation des magnetisch-markierten Reagenzes von der festen Phase überwacht wird, indem die resultierende Änderung der Kraft, welche auf die feste Phase ausgeübt wird, bestimmt wird.

4. Ein Verfahren zur Differenzierung einer spezifischen Assoziation von einer nicht-spezifischen Assoziation zwischen einer festen Phase und einem magnetisch anziehbaren Marker, wobei die spezifische Assoziation verursacht wird durch eine spezifische Bindung zwischen einem ersten Bindungsglied, das an die feste Phase angeheftet ist, und einem zweiten Bindungsglied, das an einen magnetisch anziehbaren Marker in Form eines magnetisch-markierten Reagenzes angeheftet ist, wobei das Verfahren folgende Schritte umfaßt:
a) Reagieren des ersten Bindungsglieds, das an die feste Phase angeheftet ist, und des magnetisch-markierten Reagenzes, wobei sich ein Bindungspaarkomplex bildet;
b) Anlegen und Abstimmen eines magnetischen Feldes, so daß eine Kraft auf das magnetisch-markierte Reagenz wirkt, um das magnetisch-markierte Reagenz von der festen Phase zu dissoziieren;
c) Überwachen der Dissoziation des magnetisch-markierten Reagenzes von der festen Phase,
d) Reagieren der Schritte a), b) und c) in Anwesenheit eines freien Bindungspaarglieds, welches nicht an die feste Phase angeheftet ist, wobei das freie Bindungspaarglied die Bildung des Bindungspaarkomplexes verhindert; und
e) Überwachen der Differenz der Kräfte, die nötig sind, um das magnetisch-markierte Reagenz von der festen Phase in der Abwesenheit und in der Anwesenheit des freien Bindungspaarglieds zu dissoziieren, wobei die Differenz der Kräfte abhängig ist von der spezifischen Assoziation.

5. Das Verfahren gemäß Anspruch 4, worin das freie Bindungspaarglied das gleiche ist wie das erste Bindungsglied.

6. Das Verfahren gemäß Anspruch 4, worin das freie Bindungspaarglied das gleiche ist wie das zweite Bindungsglied.

## Revendications

1. Méthode de détermination de l'affinité de liaison entre un premier et un deuxième élément de liaison comprenant :
a) la réaction dudit premier et dudit second élément de liaison en formant ainsi un complexe de paire de liaison, où ledit premier élément de liaison est fixé à une phase solide et ledit second élément de liaison est fixé à un marqueur à attraction magnétique en formant ainsi un réactif à marqueur magnétique ;
b) l'application et le réglage d'un champ magnétique de telle sorte qu'une force agit sur ledit réactif à marqueur magnétique de façon à dissocier ledit réactif à marqueur magnétique de ladite phase solide ;
c) le contrôle de la dissociation dudit réactif à marqueur magnétique de ladite phase solide et
d) la détermination de ladite force nécessaire pour dissocier ledit complexe de paire de liaison, l'ampleur de ladite force dépendant de l'association entre lesdits premier et deuxième élément de liaison.

2. Méthode selon la revendication 1 dans laquelle ladite dissociation dudit réactif à marqueur magnétique de ladite phase solide est contrôlée par des moyens visuels.

3. Méthode selon la revendication 1, dans laquelle ladite dissociation dudit réactif à marqueur magnétique de ladite phase solide est contrôlée en déterminant le changement existant dans la force exercée sur ladite phase solide.

4. Méthode pour différencier une association spécifique d'une association non spécifique entre une phase solide et un marqueur à attraction magnétique, dans laquelle ladite association spécifique est due à une liaison spécifique entre un premier élément de liaison fixé à ladite phase solide et un second élément de liaison fixé à un marqueur à attraction magnétique sous forme d'un réactif à marqueur magnétique, ladite méthode comprenant les étapes consistant à :
a) faire réagir ledit premier élément de liaison fixé à ladite phase solide et ledit réactif à marqueur magnétique en formant ainsi un complexe de paire de liaison
b) appliquer et régler un champ magnétique de sorte qu'une force agisse sur ledit réactif à marqueur magnétique de façon à dissocier ledit réactif à marqueur magnétique de ladite phase solide :
c) contrôler la dissociation dudit réactif à marqueur magnétique de ladite phase solide
d) faire réagir les étapes a), b) et c) en présence d'un élément de paire de liaison libre non fixé à ladite phase solide, l'élément de paire de liaison libre inhibant ainsi la formation dudit complexe de paire de liaison et
e) contrôler la différence de forces nécessaires pour dissocier ledit réactif à marqueur magnétique de ladite phase solide en l'absence et en présence dudit élément de paire de liaison libre, ladite différence de forces dépendant de ladite association spécifique.

5. Méthode selon la revendication 4, dans laquelle ledit élément de paire de liaison libre est le même que ledit premier élément de liaison.

6. Méthode selon la revendication 4, dans laquelle ledit élément de paire de liaison libre est le même que ledit second élément de liaison.
